# EUROPÄISCHE PATENTSCHRIFT

(11) **EP 2 054 088 B1**
(45) Veröffentlichungstag und Bekanntmachung des Hinweises auf die Patenterteilung: **16.03.2016**
(21) Anmeldenummer: 07818023.9
(22) Anmeldetag: 15.08.2007
(51) Int. Cl.: A61L 2/18, B08B 9/20, A61L 2/22, B08B 9/34, B08B 3/02

(54) **VORRICHTUNG ZUM BEHANDELN VON BEHÄLTERN**
DEVICE FOR THE TREATMENT OF CONTAINERS
DISPOSITIF POUR MANIPULER DES CONTENANTS

(30) Priorität: 16.08.2006 DE 102006038255
(43) Veröffentlichungstag der Anmeldung: 06.05.2009
(73) Patentinhaber: KHS GmbH, 44143 Dortmund (DE)
(72) Erfinder: KAPPEL, Steffen, 55545 Winzenheim (DE); BAUMGARTNER, Klaus, 55543 Bad Kreuznach (DE)
(86) Internationale Anmeldenummer: PCT/EP2007/007188
(87) Internationale Veröffentlichungsnummer: WO 2008/019828

(56) Entgegenhaltungen:
- EP-A- 1 281 446
- DE-A1- 4 425 219
- DE-A1- 19 824 991
- US-A- 5 409 545
- US-A1- 2003 188 769

## Beschreibung

Die Erfindung betrifft eine Vorrichtung zur Behandlung von Behältern, insbesondere zur Überkopfreinigung von Flaschen, mit einem Spritzrohr zur Beaufschlagung des Behälterinneren mit einem Desinfektions-/Reinigungsmittel, und mit einer Abdeckvorrichtung als Behälterersatz zur vorzugsweise Kreislaufführung des Desinfektions-/Reinigungsmittels im Zuge einer Anlagenreinigung.

Eine Vorrichtung zur Überkopfreinigung von Flaschen wird beispielhaft in dem Gebrauchsmuster DE 299 03 939 U1 beschrieben. Darüber hinaus kennt man gattungsgemäße Vorrichtungen aus der Praxis.

Eine weitere Reinigungsvorrichtung wurde durch die DE 44 25 219 A1 bekannt. Dieses Dokument zeigt auch den Oberbegriff des Anspruchs 1. Zur Reinigung der Behalterspulmaschme schlägt die DE 44 25 219 A1 vor, die Austrittsöffnungen der Spritzdüsen flüssigkeitsdicht an eine Umlaufleitung für die Reinigungsflüssigkeit anzuschließen. Dazu sieht diese Schrift weiterhin vor, die Austrittsdüsen mittels flexibler Schläuche mit den Austrittsdüsen zu verbinden, wobei die Austrittsöffnungen und die Enden der flexiblen Schläuche mit entsprechenden Kupplungselementen versehen sind.

Ebenfalls bekannt wurde eine Vorrichtung nach der EP 1281 446 A1. Diese Schrift befasst sich mit der Überprüfung der Prozesse bei der Reinigung von Behältern innerhalb einer Behälterreinigungsmaschine. Dazu schlägt diese Schrift die Verwendung eines mit Sensoren versehenen Behälters vor, welcher einen Reinigungsdurchlauf durchläuft und dabei alle relevanten Temperaturen aufzeichnet.

Ebenfalls bekannt wurden Vorrichtungen nach der US 5,409,545 und nach der US 2003/0188769 A1. Diese Schriften befassen sich mir der Reinigung von Behältern durch aus Düsen austretende Flüssigkeiten. Dazu sehen diese Schriften vor, dass der gesamte Reinigungsprozess innerhalb eines geschlossenen Gehäuses abläuft, wozu die Reinigungsdüsen auch innerhalb des geschlossenen Gehäuses angeordnet sind. Zur Reinigung der Reinigungsdüsen wiederum werden diese mit einer Reinigungsflüssigkeit gespült, welche ebenfalls aus den Düsenöffnungen austritt.

Da sich aber nun die Reinigungsdüsen innerhalb eines geschlossenen Gehäuses befinden, ist bei ihrer Reinigung keine Abdeckung der Düsenöffnungen erforderlich.

Der Reinigung von Behältern und hier insbesondere von Flaschen vor ihrem Befüllen kommt eine besondere Bedeutung zu, um beispielsweise Staub, Schmutz, Kunststoffpartikel etc. aus den Flaschen zu entfernen. Dazu werden die Flaschen vorteilhaft in eine Überkopfposition gebracht und wird das Innere der Flaschen bzw. das Behälterinnere in dieser Überkopfposition mit Hilfe von Spülflüssigkeit gereinigt bzw. "gerinst", wie dieser Vorgang im Fachjargon bezeichnet wird. Durch die Überkopfposition der Flaschen bzw. Behälter wird sichergestellt, dass das eingefüllte (gasförmige und/oder flüssige) Desinfektions-/Reinigungsmittel mit darin aufgenommenem Staub, Schmutz, Kunststoffpartikeln etc. den jeweiligen Behälter bzw. die Flasche schwerkraftunterstützt verlässt und auch verlassen kann.

Zu diesem Zweck wird das betreffende Desinfektions-/Reinigungsmittel meistens mit Hilfe einer kopfseitig des Spritzrohres vorgesehenen Düse in das Behälterinnere eingesprüht oder eingespritzt und gleichsam im Kreislauf entlang des Behälterbodens über die Behälterwandungen und schließlich die Mündung geführt, von wo aus es den jeweiligen Behälter respektive die Flasche wieder verlässt. Um das solchermaßen beispielsweise mit Schmutzpartikeln angereicherte Desinfektions-/Reinigungsmittel aufzufangen, findet sich unterhalb des Spritzrohres in der Regel eine Auffangwanne.

In neuerer Zeit wird zunehmend die so genannte kaltaseptische Abfüllung durchgeführt. Hierbei kommt insbesondere zur Reinigung der Behälterbehandlungs- oder Füllanlagen ein Desinfektions-/Reinigungsmittel zum Einsatz, welches nicht nur die bereits angesprochenen etwaigen Schmutzpartikel von den Oberflächen der Behälterbehandlungsmaschinen aufnimmt, sondern zugleich dort unter Umständen vorhandene Keime abtötet. Das findet üblicherweise bei geringen Temperaturen (Raumtemperatur) statt, wenngleich die Erfindung selbstverständlich auch eine Beaufschlagung mit beispielsweise Heißdampf abdeckt.
Um bei der Reinigung der Anlage diese nicht mehr als unbedingt erforderlich mit dem verwendeten, teilweise gesundheitlich nicht völlig unbedenklichen Mitteln zu benetzen, ist nun zusätzlich eine Abdeckvorrichtung als Flaschenersatz bzw. Behälterersatz vorgesehen. Das heißt, die Abdeckvorrichtung kommt alternativ als Ersatz für eine Flasche oder einen Behälter im Zuge der Anlagenreinigung zum Einsatz. Dabei wird das Spritzrohr wie üblich betrieben und stößt an seiner Düse das zur Reinigung der Anlage verwendete Desinfektions-/Reinigungsmittel aus, welches jetzt jedoch nicht ins Behälterinnere eindringt, sondern vielmehr gegen die Abdeckvorrichtung prallt und von hier aus das Spritzrohr, dessen Düse sowie etwaige Halte- und Schwenkvorrichtungen für den Behälter bzw. die Flasche beaufschlagt. Dadurch werden diese Anlagenbestandteile gereinigt respektive desinfiziert, wobei wiederum das mit etwaigen Schmutzpartikeln und/oder Keimen angereicherte Desinfektions-/Reinigungsmittel von der unterhalb angeordneten Auffangwanne gesammelt wird. Gleichzeitig werden durch diese Vorgehensweise auch die Zufuhr- oder Abfuhrleitungen des Rinsmediums gereinigt oder desinfiziert.

Um die Abdeckvorrichtung aus ihrer Ruheposition in eine Arbeitsposition zu überführen, in welcher sie als Behälterersatz vorzugsweise zur Kreislaufführung des Desinfektions-/Reinigungsmittels im Zuge der Anlagenreinigung fungiert, wird im aus der Praxis bekannten Stand der Technik ein manueller Schwenkvorgang initiiert. Dieser ist mit dem grundsätzlichen Problem behaftet, dass jeder manuelle Eingriff (erneut oder erstmals) Keime in die Anlage respektive Reinigungsanlage eintragen kann. Damit wird die an sich vorteilhafte Reinigungsroutine mit Hilfe der Abdeckvorrichtung gestört oder doch zumindest beeinträchtigt, insbesondere bei Anlagen zur kaltaseptischen Abfüllung wird durch diesen manuellen Eingriff eine langwierige Reinigungsprozedur erforderlich. Hier setzt die Erfindung ein.

Der Erfindung liegt das technische Problem zugrunde, eine Vorrichtung zur Behandlung von Behältern der eingangs beschriebenen Ausführungsform so weiter zu entwickeln, dass die Reinigungswirkung verbessert ist und insbesondere weitgehende Keimfreiheit erreicht wird, bzw. einmal erreichte Keimfreiheit durch vermiedene manuelle Eingriffe länger aufrechterhalten wird, wobei das aus den Spritzdüsen austretende Reinigungs- oder Desinfektionsmittel zur Reinigung weiterer Anlagenteile verwendet werden soll.

Zur Lösung dieser technischen Problemstellung ist eine Vorrichtung zur Behandlung von Behältern, insbesondere zur Überkopfreinigung von Flaschen gemäß Anspruch 1 vorgesehen.

Das heißt, erfindungsgemäß erfolgt das Überführen der Abdeckvorrichtung von ihrer Ruheposition in die Arbeitsposition und zurück nicht mehr manuell, sondern vielmehr motorisch. Auf diese Weise wird der Mensch als potentielle (weitere) Keimquelle bei der Behandlung der Behälter und insbesondere der Überkopfreinigung von Flaschen im Bereich der kaltaseptischen Abfüllung ausgeklammert. Denn der Wechsel von der Ruheposition zur Arbeitsposition kann von einem im Vergleich zu der Vorrichtung entfernten Platz aus initiiert werden, indem beispielsweise ein oder mehrere Antriebsmotoren angesteuert werden. Insbesondere ist die Abdeckvorrichtung an einen um eine Drehachse schwenkbaren Hebel angeschlossen, bei dem es sich vorteilhaft um einen Einarmhebel handeln kann. In diesem Fall findet sich die Drehachse endseitig des Einarmhebels. Selbstverständlich sind auch Konstruktionen mit Zweiarmhebel denkbar, bei welchem an einem Ende die Abdeckvorrichtung und an einem anderen Ende beispielsweise ein Gegengewicht platziert werden.

In jedem Fall greift der Antriebsmotor vorteilhaft an der Drehachse für den schwenkbaren Hebel an oder definiert die Drehachse selber. Dabei ist der Antriebsmotor regelmäßig als Rotationsmotor ausgeführt. Im erstgenannten Fall mag der Antriebsmotor über beispielsweise ein Transmissionsmittel an die Drehachse angreifen und für das Schwenken des Hebels sorgen. Bei der letztgenannten Variante kann der schwenkbare Hebel unmittelbar auf eine Ausgangswelle des Antriebsmotors aufgesteckt sein, welche dadurch die Drehachse definiert.

Es hat sich ergänzend bewährt, wenn die Abdeckvorrichtung in Bezug auf die kopfseitig des Spritzrohres vorgesehene Düse abstandsveränderlich bzw. höhenveränderlich ausgebildet ist. Dadurch lässt sich dem jeweils angewandten Desinfektions-/Reinigungsmittel und dessen Druck und/oder Fließgeschwindigkeit beim Austritt aus der Düse Rechnung tragen. Wenn beispielsweise die Austrittsgeschwindigkeit des Desinfektions-/Reinigungsmittels hoch ist und ein scharfer Strahl vorliegt, wird man mit relativ großem Abstand der Abdeckvorrichtung arbeiten, um ein Umherspritzen des Desinfektions-/Reinigungsmittels bei der Anlagenreinigung zu vermeiden oder doch zumindest so gering wie möglich einzustellen. In anderen Fällen kann man mit einem geringeren Abstand arbeiten.

Erfindungsgemäß ist die Abdeckvorrichtung insgesamt bogenförmig ausgebildet und darüber hinaus mit wenigstens einem in Richtung auf die Auffangwanne gerichteten Bogenschenkel ausgebildet. Tatsächlich sorgt dieser wenigstens eine Bogenschenkel dafür, dass das Desinfektions-/Reinigungsmittel gezielt von der Abdeckvorrichtung auf eine etwaige Schwenkvorrichtung für den Behälter, einen Haltering, die Düse des Spritzrohres, das Spritzrohr und evtl. weitere Anlagenteile gerichtet wird, die auf diese Weise die gewünschte Reinigung/Desinfektion erfahren.

Es hat sich bewährt, die Abdeckvorrichtung im Querschnitt im Wesentlichen umgekehrt U-förmig mit einer Basis und wenigstens zwei seitlichen Begrenzungsschenkeln auszugestalten. Meistens trifft das aus der Düse des Spritzrohres austretende Desinfektions-/Reinigungsmittel auf die Basis auf und wird entlang der seitlichen Begrenzungsschenkel geführt. In weiterer Ausgestaltung kann die Abdeckvorrichtung im Querschnitt bis auf eine Einführöffnung für die Düse des Spritzrohres geschlossen ausgebildet sein. Dann wird man die Abdeckvorrichtung meist auch mit einer Rücklaufleitung für das Desinfektions-/Reinigungsmittel ausrüsten, um insgesamt eine geschlossene Kreislaufführung des Desinfektions-/Reinigungsmittels zu erreichen. Das ist selbstverständlich nicht zwingend.

Im Ergebnis wird eine Vorrichtung zur Behandlung von Behältern, insbesondere zur Überkopfreinigung von Flaschen vorgestellt, die sich durch besondere Keimfreiheit auszeichnet. Das wird im Kern dadurch erreicht, dass sämtliche Reinigungssequenzen maschinell durchgeführt und initiiert werden, ohne dass ein menschlicher Eingriff erforderlich wäre. Eine Bedienperson wird vielmehr erst und nur dann tätig, wenn beispielsweise beschädigte Behälter den Betrieb stören. Sämtliche Reinigungsvorgänge werden dagegen praktisch mannlos und maschinell sowie folglich praktisch keimfrei durchgeführt. Hierin sind die wesentlichen Vorteile zu sehen.

Im Folgenden wird die Erfindung anhand einer lediglich ein Ausführungsbeispiel darstellenden Zeichnung näher erläutert; es zeigen:

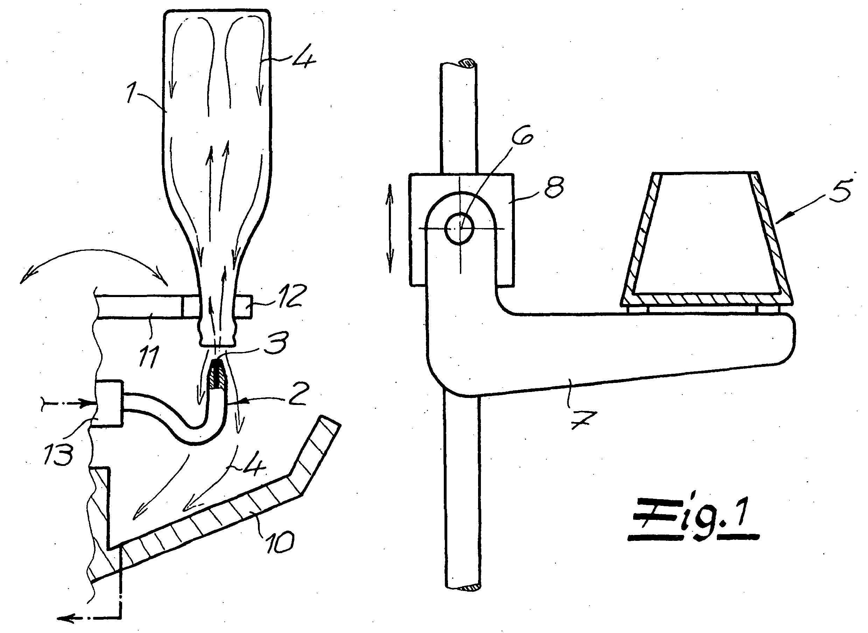

- Fig. 1: eine Vorrichtung zur Behandlung von Behältern schematisch,

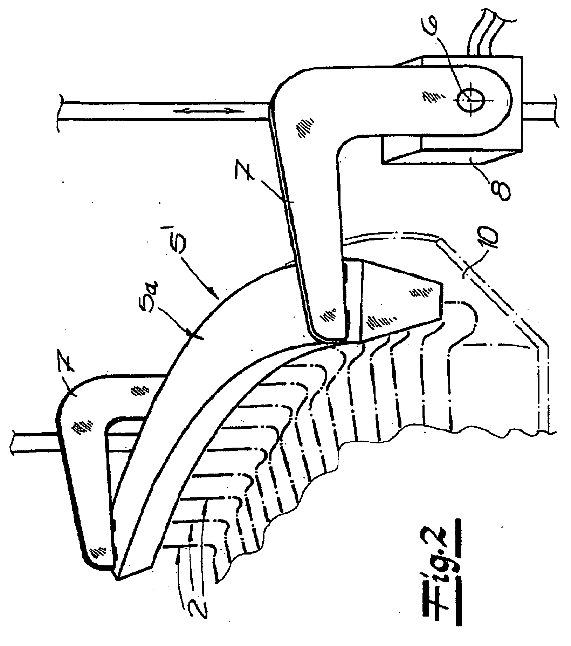

- Fig. 2: die Abdeckvorrichtung in einer Übersicht und
- Fig. 3: einen Ausschnitt aus Fig. 1 mit der Abdeckvorrichtung in Arbeitsposition (durchgezogen) und Ruheposition (gestrichelt),
- Fig. 4: eine Variante der Vorrichtung mit abgewandelter Abdeckvorrichtung.

In den Figuren ist eine Vorrichtung zur Behandlung von Behältern 1 dargestellt. Bei den Behältern 1 handelt es sich vorliegend und nicht einschränkend um Flanschen Die Vorrichtung verfügt über ein Spritzrohr 2 mit einer endseitigen Düse 3. Mit Hilfe des Spritzrohres 2 wird das Behälterinnere bzw. Flascheninnere mit einem Desinfektions-/Reinigungsmittel 4 zum Spülen und Reinigen sowie Entkeimen beaufschlagt.

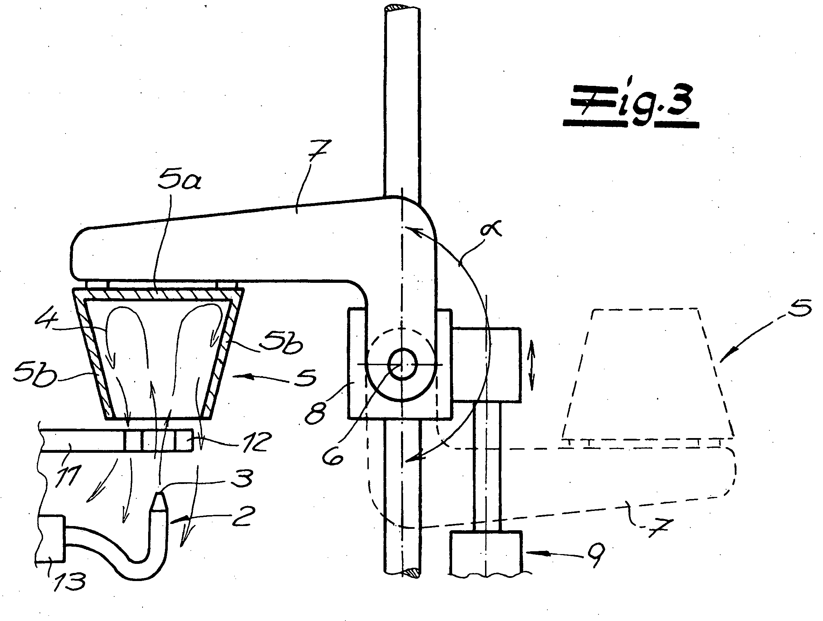

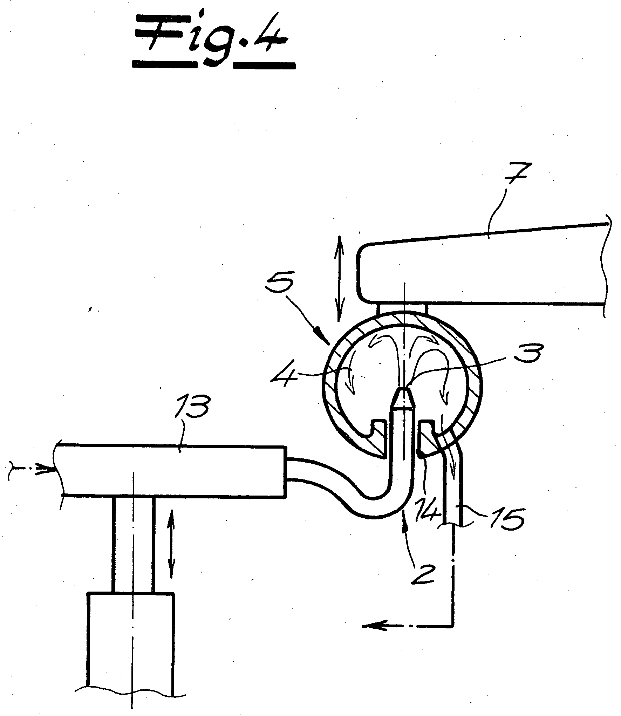

Darüber hinaus erkennt man eine Abdeckvorrichtung 5, die an einen um eine Drehachse 6 schwenkbaren Hebel 7 angeschlossen ist. Die Abdeckvorrichtung 5 fungiert als Behälterersatz bzw. Flaschenersatz und dient - wie in den Fig. 3 und 4 dargestellt - zur Kreislaufführung des Desinfektions-/Reinigungsmittels 4 im Zuge einer Anlagenreinigung. Das ist jedoch nicht zwingend.

Erfindungsgemäß wird die fragliche Abdeckvorrichtung 5 motorisch verschwenkt, und zwar von einer in der Fig. 3 gestrichelt dargestellten Ruheposition in eine durchgezogen gezeigte Arbeitsposition und zurück. Zu diesem Zweck ist ein Antriebsmotor 8 vorgesehen, der vorliegend als Drehantrieb ausgebildet ist bzw. als rotierender Antriebsmotor 8 und unmittelbar an der Drehachse 6 des schwenkbaren Hebels 7 angreift bzw. die Drehachse 6 mit seiner Abtriebswelle definiert.

Bei dem schwenkbaren Hebel 7 handelt es sich um einen Einarmhebel, an dem endseitig der Antriebsmotor 8 und die Drehachse 6 vorgesehen sind. Beim Wechsel von der Ruheposition in die Arbeitsposition und zurück überstreicht der schwenkbare Hebel 7 und folglich die Abdeckvorrichtung insgesamt einen Winkel □ von angenähert 180°. Selbstverständlich sind auch andere Winkel □ denkbar (vgl. Fig. 3).

Anhand der Fig. 3 erkennt man, dass die Abdeckvorrichtung 5 in Bezug auf die Düse 3 und folglich das Spritzrohr 2 insgesamt abstandsveränderlich bzw. höhenveränderlich ausgebildet ist. Dazu ist die Abdeckvorrichtung 5 ergänzend an einen Stelltrieb 9 angeschlossen, der vorliegend als Pneumatikzylinder ausgeführt sein mag. Der Stelltrieb 9 ist an dem schwenkbaren Hebel 7 befestigt oder an einer in der Fig. 3 dargestellten separaten Haltevorrichtung.

Die Abdeckvorrichtung 5 ist insgesamt bogenförmig ausgestaltet und verfügt über wenigstens einen Bogenschenkel 5', welcher in Richtung auf eine Auffangwanne 10 verläuft. Tatsächlich befindet sich die Abdeckvorrichtung 5 in ihrer Arbeitsposition oberhalb des Spritzrohres 2, an welches sich darunter die fragliche Auffangwanne 10 anschließt. Man erkennt, dass das aus der Düse 3 des Spritzrohres 2 austretende Desinfektions-/Reinigungsmittel 4 in der Arbeitsposition gegen eine Basis 5a der im Querschnitt im Wesentlichen umgekehrt U-förmig ausgeführten Abdeckvorrichtung 5 trifft. Neben dieser Basis 5a verfügt die Abdeckvorrichtung 5 im Querschnitt über zwei seitliche Begrenzungsschenkel 5b, an welchen das Desinfektions-/Reinigungsmittel 4 - ausgehend von der Basis 5a - in im Wesentlichen zwei Kreisbögen weitergeführt wird.

Fußseitig der Begrenzungsschenkel 5b verlässt das Desinfektions-/Reinigungsmittel 4 die Abdeckvorrichtung 5 und beaufschlagt im Ausführungsbeispiel und nicht einschränkend eine Schwenkvorrichtung 11 für die jeweiligen Flaschen 1 zur Einnahme ihrer Überkopfstellung, zusätzlich einen Haltering 12 für die Mündung der Flasche 1 sowie selbstverständlich die Düse 3 und das Spritzrohr 2 im Ganzen sowie eine etwaige Halterung 13 für das Spritzrohr 2. Auf diese Weise kommt es zu der bereits angesprochenen Anlagenreinigung, wobei mit Hilfe der Abdeckvorrichtung 5 als Behälterersatz das Desinfektions-/Reinigungsmittel 4 im Kreislauf geführt wird. Das ist jedoch nicht zwingend.

Bei der Variante nach Fig. 4 ist die Abdeckvorrichtung 5 im Querschnitt bis auf eine Einführöffnung 14 für die Düse 3 des Spritzrohres 2 geschlossen ausgebildet. Um das Desinfektions-/Reinigungsmittel 4 zurückzuführen ist an dieser Stelle eine Rücklaufleitung 15 realisiert, welche an die Abdeckvorrichtung 5 angeschlossen ist. Man erkennt, dass in diesem Fall und auch ansonsten das Spritzrohr 2 höhenvariabel ausgeführt ist und zu diesem Zweck eine Höhenverstellung für die Halterung 13 aufweist.

## Patentansprüche

1. Vorrichtung zur Behandlung von Behältern (1), insbesondere zur Überkopfreinigung von Flaschen (1), mit einem Spritzrohr (2) zur Beaufschlagung des Behälterinneren mit einem Desinfektions-/Reinigungsmittel (4), und mit einer Abdeckvorrichtung (5) als Behälterersatz zur vorzugsweise Kreislaufführung des Desinfektions-/Reinigungsmittels (4) im Zuge einer Anlagenreinigung, mit einer Auffangwanne (10), **dadurch ge kennzeichnet**, dass die Abdeckvorrichtung (5) motorisch verschwenkbar aus einer Ruheposition in eine Arbeitsposition und zurück ausgebildet ist, und dass die Abdeckvorrichtung (5) insgesamt bogenförmig ausgebildet ist und mit wenigstens einem, im Arbeitsposition der Abdeckvorrichtung (5) in Richtung der Auffangwanne (10) verlaufenden Bogenschenkel (5') ausgebildet ist, an dessen Fußseite das Desinfektions- oder Reinigungsmittel die Abdeckvorrichtung (5) verlässt und auf eine etwaige Schwenkvorrichtung für den Behälter, einen Haltering, die Düse des Spritzrohres, das Spritzrohr und eventuell weitere Anlagenteile gerichtet wird.

2. Vorrichtung nach Anspruch 1, **dadurch gekennzeichnet, dass** die Abdeckvorrichtung (5) an einen um eine Drehachse (6) schwenkbaren Hebel (7), insbesondere Einarmhebel, angeschlossen ist.

3. Vorrichtung nach Anspruch 1 oder 2, **dadurch gekennzeichnet, dass** ein an der Drehachse (6) angreifender oder die Drehachse (6) definierender Antriebsmotor (8) vorgesehen ist.

4. Vorrichtung nach einem der Ansprüche 1 bis 3, **dadurch gekennzeichnet, dass** die Abdeckvorrichtung (5) in Bezug auf eine Düse (3) des Spritzrohres (2) abstandsveränderlich ausgebildet ist.

5. Vorrichtung nach einem der Ansprüche 1 bis 4, **dadurch gekennzeichnet, dass** die Abdeckvorrichtung (5) im Querschnitt im Wesentlichen umgekehrt U-förmig mit einer Basis (5a) und wenigstens zwei seitlichen Begrenzungsschenkeln (5b) ausgeführt ist.

6. Vorrichtung nach einem der Ansprüche 1 bis 5, **dadurch gekennzeichnet, dass** die Abdeckvorrichtung (5) im Querschnitt bis auf eine Einführöffnung (14) für die Düse (3) des Spritzrohres (2) geschlossen ausgebildet ist.

7. Vorrichtung nach einem der Ansprüche 1 bis 6, **dadurch gekennzeichnet, dass** das Spritzrohr (2) an eine höhenveranderliche Haltevorrichtung (13) angeschlossen ist.

8. Vorrichtung nach einem der Anspüche 1-7 **dadurch gekennzeichnet, dass** die Abdeckvorrichtung (5) eine Rücklaufleitung (15) für das Desinfektions-/Reinigungsmittel (4) aufweist.

## Claims

1. Device for the treatment of containers (1) especially for the overhead cleaning of bottles (1), comprising an injection pipe (2) for impinging the interior of the container with a disinfection/cleaning agent (4) and a cover element (5) as a container substitute for preferably recirculating the disinfection/cleaning agent (4) during the cleaning of the installation, with a catchment trough (10), **characterised in that** the cover element (5) is configured such as to be swivelled from an idle position to a working position and back by means of a motor, and that the cover element (5) is configured overall as bow-shaped, and with at least one bow limb (5') running in the direction of the catchment trough (10) in the working position of the cover element (5), from the foot side of said limb the disinfection or cleaning agent (4) leaves the cover element (5) and is directed onto a pivot device for the container, a retaining ring, the nozzle of the injection pipe, the injection pipe, and possibly other parts of the system.

2. Device according to claim 1, **characterised in that** the cover element (5) is connected to a lever (7), especially a single-arm lever, which can pivot about an axis of rotation (6).

3. Device according to claim 1 or 2, **characterised in that** a drive motor (8) is provided, which engages at the axis of rotation (6) or defines the axis of rotation (9).

4. Device according to any one of claims 1 to 3, **characterised in that** the cover element (5) is configured such as to have a changing spacing interval in relation to a nozzle (3) of the injection pipe (2).

5. Device according to any one of claims 1 to 4, **characterised in that** the cover element (5) is configured in cross-section in an essentially inverted U-shape, with a base (5a) and at least two lateral delimitation limbs (5b).

6. Device according to any one of claims 1 to 5, **characterised in that** the cover element (5) is configured in cross-section as closed, except for an infeed opening (14) for the nozzle (3) of the injection pipe (2).

7. Device according to any one of claims 1 to 6, **characterised in that** the injection pipe (2) is connected to a height-adjustable retaining device (13).

8. Device according to any one of claims 1 to 7, **characterised in that** the cover element (5) comprises a return flow line (15) for the disinfection/cleaning agent (4).

## Revendications

1. Dispositif servant à manipuler des contenants (1), en particulier servant à nettoyer en position renversée des bouteilles (1), comprenant un tuyau de pulvérisation (2) servant à soumettre l'intérieur de contenant à un agent de désinfection/de nettoyage (4), et comprenant un dispositif de recouvrement (5) faisant office d'un substitut de contenant servant de préférence à mettre en circulation l'agent de désinfection/de nettoyage (4) au cours d'un nettoyage d'équipement, comprenant une cuve de collecte (10), **caractérisé en ce que** le dispositif de recouvrement (5) est réalisé de manière à pouvoir être pivoté de manière motorisée depuis une position de repos dans une position de travail et inversement, et **en ce que** le dispositif de recouvrement (5) est réalisé globalement de manière à présenter une forme d'arc et est réalisé, en position de travail du dispositif de recouvrement (5), avec au moins une branche formant un arc (5') s'étendant en direction de la cuve de collecte (10), au niveau du côté de base de laquelle l'agent de désinfection ou de nettoyage quitte le dispositif de recouvrement (5), et est orienté sur un dispositif de pivotement éventuel pour le contenant, une bague de maintien, la buse du tuyau de pulvérisation, le tuyau de pulvérisation, et éventuellement d'autres pièces d'équipement.

2. Dispositif selon la revendication 1, **caractérisé en ce que** le dispositif de recouvrement (5) est raccordé au niveau d'un levier (7) pouvant pivoter autour d'un axe de rotation (6), en particulier au niveau d'un levier à un bras unique.

3. Dispositif selon la revendication 1 ou 2, **caractérisé en ce qu'**un moteur d'entraînement (8) venant en prise au niveau de l'axe de rotation (6) ou définissant l'axe de rotation (6) est prévu.

4. Dispositif selon l'une quelconque des revendications 1 à 3, **caractérisé en ce que** le dispositif de recouvrement (5) est réalisé selon une distance variable par rapport à une buse (3) du tuyau de pulvérisation (2).

5. Dispositif selon l'une quelconque des revendications 1 à 4, **caractérisé en ce que** le dispositif de recouvrement (5) est réalisé de manière à présenter essentiellement une forme de U inversé dans la section transversale avec une base (5a) et au moins deux branches de délimitation (5b) latérales.

6. Dispositif selon l'une quelconque des revendications 1 à 5, **caractérisé en ce que** le dispositif de recouvrement (5) est réalisé de manière fermée dans la section transversale jusqu'à une ouverture d'introduction (14) pour la buse (3) du tuyau de pulvérisation (2).

7. Dispositif selon l'une quelconque des revendications 1 à 6, **caractérisé en ce que** le tuyau de pulvérisation (2) est raccordé à un dispositif de maintien (13) à hauteur variable.

8. Dispositif selon l'une quelconque des revendications 1 à 7, **caractérisé en ce que** le dispositif de recouvrement (5) présente un conduit de reflux (15) pour l'agent de désinfection/de nettoyage (4).
